# EUROPEAN PATENT SPECIFICATION

(11) **EP 3 262 550 B1**
(45) Date of publication and mention of the grant of the patent: **10.04.2019**
(21) Application number: 16712072.4
(22) Date of filing: 23.02.2016
(51) Int. Cl.: G06F 19/00

(54) **DETECTION OF MISSING FINDINGS FOR AUTOMATIC CREATION OF LONGITUDINAL FINDING VIEW**
DETEKTION VON FEHLENDEN BEFUNDEN ZUR AUTOMATISCHEN ERZEUGUNG DER LÄNGSANSICHT VON BEFUNDEN
DÉTECTION DE RÉSULTATS MANQUANTS POUR LA CRÉATION AUTOMATIQUE D'UNE VUE LONGITUDINALE DE RÉSULTATS

(30) Priority: 25.02.2015 US 201562120394 P
(43) Date of publication of application: 03.01.2018
(73) Proprietor: Koninklijke Philips N.V., 5656 AE Eindhoven (NL)
(72) Inventor: MABOTUWANA, Thusitha Dananjaya De Silva, 5656 AE Eindhoven (NL); SEVENSTER, Merlijn, 5656 AE Eindhoven (NL)
(74) Representative: Beck, Christopher Andrew
(86) International application number: PCT/IB2016/050958
(87) International publication number: WO 2016/135619

(56) References cited:
- WO-A1-2012/017418
- WO-A1-2014/030092
- WO-A1-2014/155273
- US-A1- 2014 006 926
- BUI A A T ET AL: "TimeLine: Visualizing Integrated Patient Records", IEEE TRANSACTIONS ON INFORMATION TECHNOLOGY IN BIOMEDICINE, IEEE SERVICE CENTER, LOS ALAMITOS, CA, US, vol. 11, no. 4, 1 July 2007 (2007-07-01), pages 462-473, XP011186857, ISSN: 1089-7771, DOI: 10.1109/TITB.2006.884365

## Description

### FIELD OF THE INVENTION

The following generally relates to medical longitudinal tracking systems, and is described with particular application to tracking of lesions or solid tumors.

### BACKGROUND OF THE INVENTION

Lesion tracking is used to evaluate an effectiveness of a treatment over time and to evaluate how lesions, such as cancerous tumors, respond to treatments. Decisions are made by healthcare practitioners according to guidelines, such as Response Evaluation Criteria in Solid Tumors (RECIST), based on changes in lesions identified as malignant over time. Those decisions can alter treatment for a particular patient based on longitudinally tracked measurements of individual lesions.

Measurements of lesions are taken by a healthcare professional from medical images of the patient obtained by an imaging device or scanner, such as a Computed Tomography (CT) scanner. The healthcare professional or radiologist evaluates the medical images to determine a type of lesion, and with the measurements narrates a report, which describes characteristics of the lesions at the time of the images, e.g. at a treatment interval, and can include comparisons with prior measurements to identify or highlight changes. A healthcare practitioner, such as a research clinical associate, typically will intercept the report and enter select index lesions in a tracking system such as a spreadsheet program.

Lesion tracking systems are typically optional. That is, a radiologist can narrate and deliver a report without entering measurements into the tracking system. With multi-organizational practices and time pressures, subsequent practitioners do not revisit prior studies to obtain missing measurements. With missing measurements the utility of the lesion tracking is diminished. Longitudinal tracking computations cannot be made with missing measurements. A typical systems approach of requiring entry of values is not practical. For example, enforcement of data entry calls for multi-organizational support and enforcement, and raises system integration issues across organizations. Another typical systems approach uses extract, transform and load (ETL) programs used in data loads, such as often used in Data Mining approaches. Data loads are typically performed with structured data at predetermined intervals. Assumptions are made about the data to facilitate loading without professional review of data values. With the reports initiated by different radiology sources, departments, or even different organizations, managing even the sourcing of the reports is a challenge.

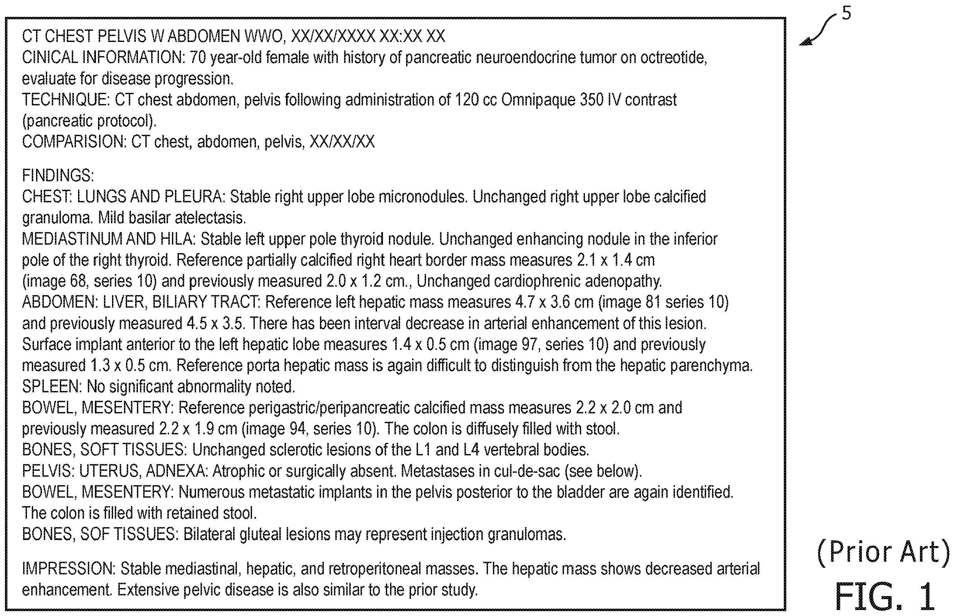

Radiology reports are typically submitted and/or stored electronically. An example is shown in Figure 1. A separate report is issued for each evaluation point, e.g. a date typically corresponding to a treatment interval of the patient. Reports include unstructured narrative 5, which include headings, and typically compare current and prior measurements of each of the lesions. The format, organization, unit of measurement and the description of each lesion can vary from one report to the next for the same patient, vary from patient to patient, and vary by healthcare practitioner and/or healthcare provider organization.

Document WO 2014/155273 discloses a system for generating a context driven summary review of medical findings without a longitudinal tracking system comprising a lesion tracking unit as defined in the appended claims. Document WO 2014/030092 discloses a system for extracting context information form a current medical image. Document WO2012017418 discloses a system for aiding report authoring in a medical context.

### SUMMARY OF THE INVENTION

Aspects described herein address the above-referenced problems and others.

In one aspect, a longitudinal tracking system includes a lesion tracking unit and a display device. In response to a received patient identifier, the lesion tracking unit constructs a display of characteristic information for at least one longitudinally tracked lesion retrieved according to the patient identifier, and an identifier of at least one missing measurement determined by comparing a temporal identifier of retrieved reports with the characteristic information, and each report includes a narrative with measurements of at least one reported lesion for the patient identifier. The display device displays the constructed display of the characteristic information for each longitudinally tracked lesion, and the indicator of the at least one missing measurement.

In another aspect, a method of longitudinal tracking includes displaying on a display device, in response to a received patient identifier, a constructed display of characteristic information for at least one longitudinally tracked lesion retrieved according to the patient identifier, and an indicator of at least one missing measurement determined by comparing a temporal identifier of retrieved reports with the characteristic information, and each report includes a narrative with measurements of at least one reported lesion for the patient identifier.

In another aspect, a longitudinal tracking system includes one or more data processors in response to a received patient identifier, display on a display device a constructed display of characteristic information for at least one longitudinally tracked lesion retrieved according to the patient identifier, and an indicator of at least one missing measurement determined by comparing a temporal identifier of retrieved reports with the characteristic information, and each report includes a narrative with measurements of at least one reported lesion for the patient identifier. The one or more data processors in response to receiving an indication to find the at least one missing measurement, update the display of the characteristic information for each longitudinally tracked lesion with found measurements corresponding to the at least one missing measurement and the found measurements found in the report narratives.

In one instance finding missing measurements provide more complete longitudinal information about tracked lesions. More complete longitudinal information aides healthcare practitioner review of the longitudinal information and provides for more informed decision making concerning patients with tracked lesions. In one instance, continued optional entry of measurements continues.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.
FIGURE 1 shows an example prior art radiology report for a patient with lesions.
FIGURE 2 schematically illustrates an embodiment of a detection of missing findings with automatic creation of longitudinal finding view system.
FIGURE 3 shows an example of three narrative report fragments with different temporally related measurements.
FIGURE 4 shows an example display of the detection of missing findings with automatic creation of longitudinal finding view system.
FIGURE 5 flowcharts an embodiment of a method of detecting missing findings with automatic creation of a longitudinal finding view.

### DETAILED DESCRIPTION OF EMBODIMENTS

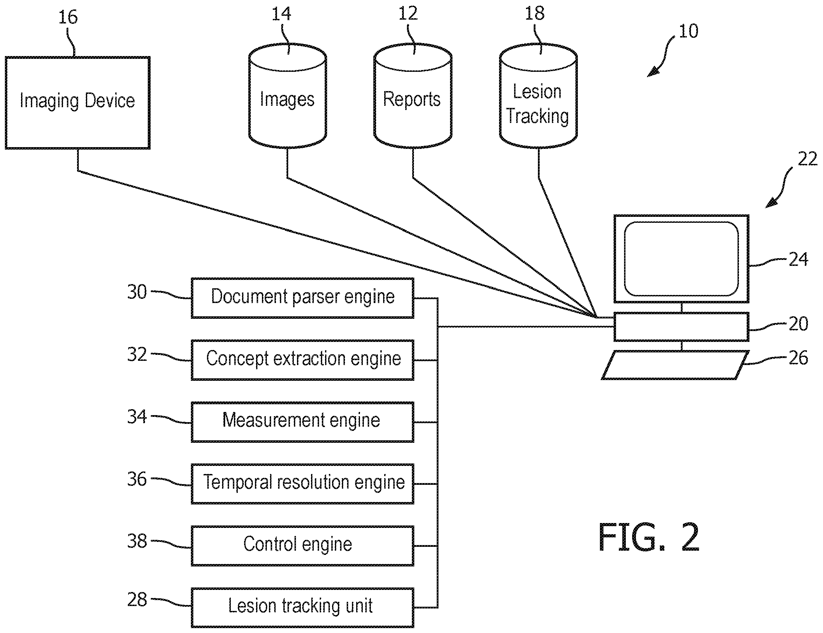

Initially referring to FIGURE 2, an embodiment of a detection of missing findings with automatic creation of longitudinal finding view system 10 is schematically illustrated. A report data store 12 includes radiology reports reporting lesions. The example report narrative 5 is shown in Figure 1. Each report includes a patient identification and a report temporal identifier of the date of lesion examination or imaging, e.g. a date of an examination from an image of the patient. The report is generated based on measurements of patient images 14 from an imaging device 16, such as a CT scanner, a magnetic resonance (MR) scanner, a positron emission tomography (PET) scanner, a single proton emission computed tomography (SPECT), a hybrid, a combination and the like. The patient images 14 can be stored in a Picture Archiving and Communication System (PACS), departmental Radiology Information System (RIS), Hospital Information System (HIS), and the like. The reports 12 can be stored in the same system, or in a separate data store.

A lesion tracking data store 18 stores characteristic information about lesions identified for each patient. For each lesion, characteristics of the lesion, such as a description and measurements, are stored with measurements stored according to each temporally indicated period measured. For example, measurements of a hypodense liver lesion measured in a CT image slice with a largest length of 14 mm and a second length orthogonal to the largest length of 13.9 mm are stored with a first date. For a second date, the measurements are 16.9 mm and 15.2 mm respectively. The data recorded for each patient can include multiple lesions.

The data stores 12, 14, 18 can include data organization, such as a file system, a database management system, an element definition, an object definition and the like. The data store includes local and/or remote non-transitory storage medium, such as disk storage, solid state storage, server storage, local storage, cloud storage and the like. The data stores are communicatively connected to at least one data processor 20, such as an electronic data processor, optical data processor, microprocessor, computer processor, and the like. The data processor 20 comprises a computing device 22, such as a desktop computer, laptop computer, portable computing device, smartphone, body worn computing device, or as a distributed computing device, such as a computing device served by a web server or other type of application server. The computing device 22 includes a display device 24 and one or more input devices 26, such as a keyboard, mouse, microphone, and the like. The display device 24 and the input device 26 can be combined, such as a touch screen device.

A 'display' or 'display device' as used herein encompasses an output device or a user interface adapted for displaying images or data. A display may output visual, audio, and or tactile data. Examples of a display include, a computer monitor, a television screen, a touch screen, tactile electronic display, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, body-mounted displays, and the like.

A lesion tracking unit 28, in response to receiving a patient identifier retrieves the characteristic information about the tracked lesions from the lesion tracking data store 18 for the identified patient and for the temporal identifiers stored, e.g. dates of tracked prior examination measurements. The lesion tracking unit 28 receives the reports from the reports data store 12 and identifies the report temporal identifier. The report patient identification and the report temporal identifier are located in the file descriptor or metadata and/or in the unstructured narrative of the report. For example, a file name, and/or file metadata can include both the patient identifier and date of the imaging examination.

The lesion tracking unit 28 identifies missing temporal identifiers in the tracked lesion data based on the identified report temporal identifier of each report received, e.g. a report temporal identifier not found in the tracked lesion data, or a tracked lesion temporal identifier for which no measurements exist. The lesion tracking unit 28 constructs a display of the tracked lesions including characteristic data and an identifier of each missing measurement, and displays the constructed display on the display device 24. The display can include the measurements for temporal periods which are known, e.g. present in lesion tracking data store 18.

In response to a request to find the missing measurements, such as a signal input from the input device 26 a document parser engine 30 parses reports corresponding to each missing period of one or more tracked lesions. For example, in response to a command received from the input device 26, the document parser engine 30 selects two reports: a January 1 report and a May 12 report corresponding to missing periods of two lesions, one lesion missing values from January 1 and May 12, and a second lesion missing values from the May 12 report. The document parser engine 39 parses the two reports.

The document parser engine 30 parses sections, paragraph headings, and/or sentences from the medical narrative. The document parser engine 30 can use predetermined section headers and/or paragraph headers, which facilitate processing. Section and paragraph headings are recognized and normalized to a pre-determined set. For example, a predetermined set of sections headings includes patient information, clinical information, technique, comparison, findings, and impression. In another example, paragraph headings include anatomical identifiers, such as chest, lungs and pleura, mediastinum and hila, abdomen, liver, biliary tract, spleen, bowel, bones. Section and paragraph headers can be nested or hierarchically related. For example, a report narrative includes: "LIVER, BILIARY TRACT: Probable diffuse fatty liver. Subtle hypodense soft tissue along the subcapsular portion of the liver segment 7 measures 1.1 x 2.7 cm. Previously 3.2 x 1.3 cm" is parsed by the document parser engine into sentences of "LIVER, BILIARY TRACT: Probable diffuse fatty liver," "Subtle hypodense soft tissue along the subcapsular portion of the liver segment 7 measures 1.1 x 2.7 cm." and "Previously 3.2 x 1.3 cm," "LIVER, BILIARY TRACT" are identified as a header.

The document parser engine 30 can be implemented using rule-based, machine learning, maximum entropy or other techniques using commercially available products or other products that include header recognition. The document parser engine 30 can identify the patient identifier and the report temporal identifier when part of the narrative and not available as part of the file descriptor or metadata. The document parser engine 30 can identify related images, such as the images from which the report is based.

A concept extraction engine 32 recognizes phrases in the parsed sentences and maps the phrases to an external ontology, such as SNOMED, UMLS or RadLex, using a commercially available product, such as MetaMap. For example, the labels of the lesions are recognized from the parsed sentences and mapped to the ontology. Referring to the example of the parsed report fragment, "LIVER, BILIARY TRACT" are mapped to the ontology, which indicates the information is about the LIVER and/or BILIARY TRACT. Phrases, such as "hypodense," "soft tissue," "subcapsular" and "liver segment" are mapped to the ontology referring to the mapped liver and biliary tract.

A measurement engine 34 recognizes measurements in the parsed text and associated with the mapped phrases and normalizes the recognized measurements. The measurements are recognized based on rules and/or pattern matching searching the parsed sentences as character strings for numeric values. The measurement engine 34 normalizes the measurements to a standard unit of measure. For example, measurements of the lesions in centimeters (cm) or inches (in) are converted to millimeters (mm), or other unit of measure selected for the tracked characteristics. Referring to the previous example of the parsed sentences: "...measures 1.1 x 2.7 cm" and "Previously 3.2 x 1.3 cm," the measurement engine recognizes two measurements ".1 x 2.7 cm" and "3.2 x 1.3 cm," which are normalized to 1 x 27 mm and 32 x 13 mm. The normalized unit of measure, e.g. mm, can be selectable as a system parameter, e.g. as stored in the lesion tracking data store 18. A display parameter for unit of measure can be included in configuration settings for the user and/or computing device 22.

A temporal resolution engine 36 identifies temporal periods or identifiers associated with each measurement. For example, in the parsed sentence, "Liver lesion measures 1.2 x 2.3 cm, previously measuring 0.6 x 1.2 cm," the second measurement is temporally associated with a different temporal identifier, e.g. different report, and the first measurement is associated with the report temporal identifier, e.g. current report. In one embodiment, the temporal resolution engine 36 identifies the corresponding temporal periods of images recognized by the parsing engine, e.g. an image corresponding to the examination being reported on by the current report, or a prior reference image corresponding to a prior examination and/or cross referenced report used to compare.

In one instance, the temporal resolution engine 36 includes a classifier trained to determine with which examination or study a measurement is associated. In one embodiment, the technique uses Regular Expressions (REs) with a statistical decision making layer defined by a maximum entropy model. For example, the order of the reported measurements, and accompanying words such as "previously" can be used to statistically classify the measurement as the same temporal identifier of report or a different report. In one embodiment, the temporal resolution engine 36 classifies each measurement according to a report temporal identifier, e.g. to which report a measurement corresponds.

A control engine 38 matches the temporally resolved measurements with the missing periods for each lesion. The control engine 38 matches a description or label of the reported lesion to the tracked lesions based on the ontology to identify the corresponding lesion in the tracked lesion. The control engine 38 can identify new or missing lesions, e.g. reported and not currently tracked. The control engine 38 matches or associates the measurements to the missing measurements based on the temporal resolution, i.e. the identified temporal period for a measurement corresponds to the tracked temporal period that includes missing measurements. The control engine 38 can match measurements to other measurements of other temporal identifiers for verification. For example, measurements of identified with prior temporal identifiers are compared with tracked lesion measurements to verify that the measurements are correct and/or use to confirm that the measurement corresponding to prior measurements describe the same lesion, e.g. direct match or ontological match.

The control engine 38 uses a rule based match, or a statistical method to determine the match, such as a rankings or a maximum likelihood estimate. The control engine 38 can report no match. For example, various parameters can be used to group the measurements by lesion, including volumetric similarity between measurements, the semantic similarity between the sentence(s) in which the measurements are described, whether the measurements appear in the paragraphs with the same or a similar header, and image slice information identified by the temporal resolution engine 36. In one embodiment, a similarity score associated with each grouping indicates the confidence level for each cross-report link, e.g. measurements or image referring to a prior examination.

The lesion tracking unit 28 constructs and/or revises the display of the tracked lesions to include the missing measurements matched by the control engine 38. The display is displayed by the display device 22 and can include an identifier of the added or found measurements for the missing measurements, such as bolded or high intensity highlighted values and/or a message asking for confirmation. In one embodiment, in response to input command by the input device 26, the lesion tracking unit 28, displays the report fragment with the identified measurement. In another embodiment, the response can include the image referenced in report narrative and temporally resolved by the temporal resolution engine 36.

The various engines or units 28, 30, 32, 34, 36, 38 are suitably embodied by the data processor 20 configured to execute computer readable instructions stored in a non-transitory computer readable storage medium or computer readable memory, e.g. software. The data processor 20 can also execute computer readable instructions carried by a carrier wave, a signal or other transitory medium to perform the disclosed techniques.

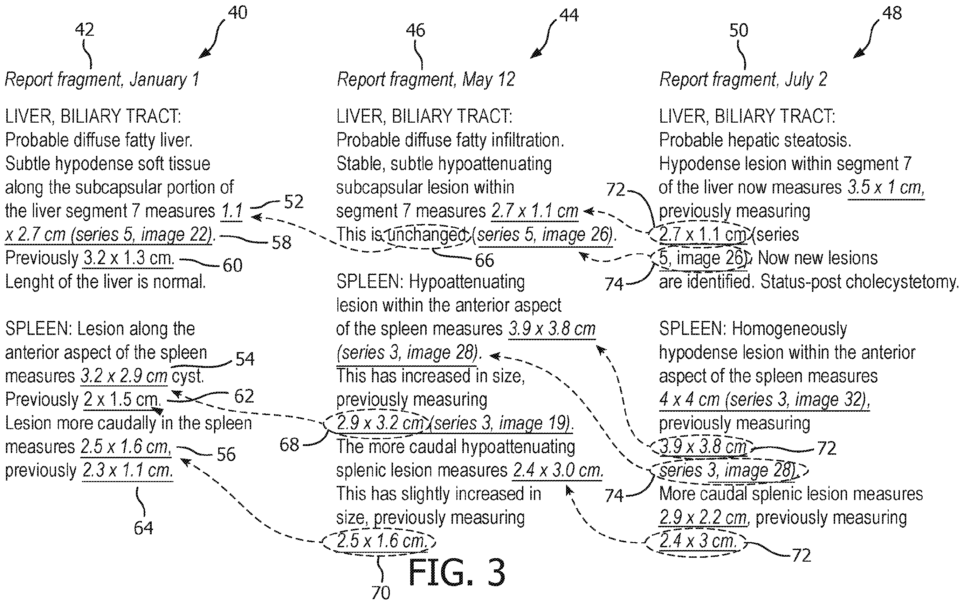

With reference to FIGURE 3, an example of three narrative report fragments with different temporally related measurements of one patient is shown. A first report fragment 40 is from a January 1 report identified with a temporal identifier 42 of January 1, a second report fragment 44 is from a May 12 report fragment with a temporal identifier 46 of May 12, and a third report fragment 48 is from a July 2 report with a temporal identifier 50 of July 2. Each report fragment includes measurements for 3 lesions, a liver lesion and two spleen lesions. Measurements and image identifiers, which are temporally related to the report temporal identifier, are underlined and italicized. Measurements and image identifiers, which are temporally related to a different report, are underlined and not italicized. In the first report fragment 40, measurements 52 of the liver lesion and measurements 54, 56 of the spleen lesion and a first image reference 58 corresponds to the report temporal identifier 42. Other measurements 60 of the liver lesion and other measurements 62, 64 of the spleen lesions correspond to a different temporal identifier, which is not shown or indicated.

An implied measurement 66 of a prior temporal identifier is shown in the second report fragment 44. The sentence, "This is unchanged," refers both to a measurement "2.7 x 1.1 cm" of the report temporal identifier 46 and to a different report measurement 52 with another temporal identifier 42. The temporal identifiers are shown as dates. The temporal identifiers can include both time and dates. Explicit measurements 68, 70 can be matched with different report measurements 54, 56. The different report measures can be used to verify the specific lesion, e.g. measurements referring to lesion in report is same as tracked lesion, and/or verify the accuracy of the measurements.

Measurements 72 can include image references 74, such as the report temporal identifier or the prior or cross-report temporal identifier. The image references 74 can also be used to verify the specific lesion and/or verify the accuracy of the measurements. The image references 74 can be used to retrieve the corresponding image from the image data store 14 and display to the healthcare practitioner the source of the measurements to confirm the found measurements correspond correctly to the missing measurements.

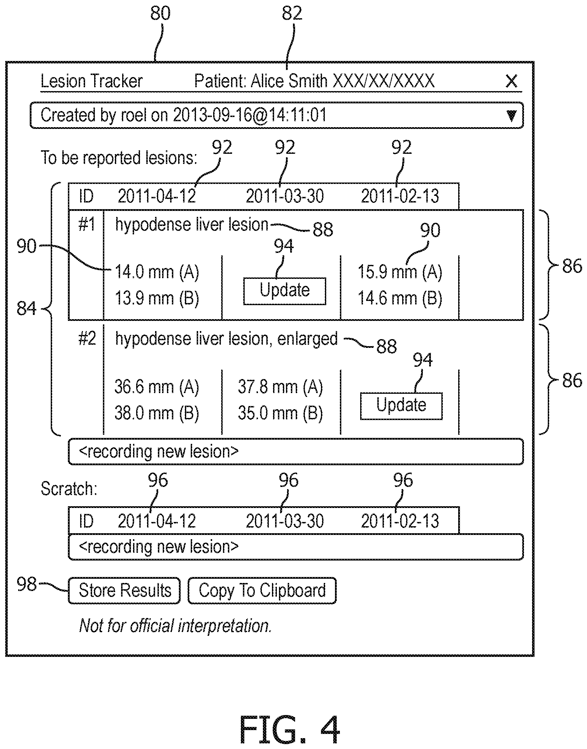

With reference to FIGURE 4, an example display 80 of the detection of missing findings with automatic creation of longitudinal finding view system 10 is shown. The display 80 includes a patient identification 82, such as a patient name and patient identifier, e.g. alphanumeric patient identifier. The display includes characteristic information 84 of tracked lesions retrieved from the lesion tracking data store 18. Each tracked lesion 86 includes a lesion identifier or label 88, and a series of measurements 90, each measurement 90 corresponding to a temporal identifier 92, e.g. date or date-time of imaging/examination. The measurement 90 can include one or more values, such as a longest length of the lesion measured in a CT slice image, and an orthogonal longest width. The tracked measurements 90 include missing measurements, which are indicated with a missing measurement indicator 94, such as a button.

The missing measurements are determined from report temporal identifiers and can be displayed as temporal identifiers 96. Additional or missing measurements and/or lesions can be manually added by a healthcare practitioner with measurements from selected images stored in a scratch area, e.g. computer memory. The temporal identifiers 96 of reports are determined from reports in the reports data store 12, and compared with the tracked temporal measurements 90 to determine that one or more temporally indicated measurements are missing. In one instance, the temporal identifiers 96 alternatively indicate measurements manually added to a scratch area by the healthcare practitioner taken from one or more images, which correspond to the temporal identifiers 96. The healthcare practitioner can find the missing measurements with an input by the input device 26, such as selecting a missing measurement indicator, e.g. selecting the "update" button. In response to receiving the input, the system finds the missing measurement from the narrative of the corresponding report based on the temporal identifier, e.g. tracked temporal identifier 92 of missing measurement identifier 94 indicating a report with a corresponding temporal identifier. The display 80 is updated with the associated or found measurements, or a new display constructed. The display can include displaying the corresponding report fragment and/or referenced image.

A confirmation identifier 98, such as a "store results" button, is invoked to confirm the associated measurements are to be stored in the lesion tracking data store 18. For example, the healthcare practitioner invokes the "store results" button using the input device 26, which sends a signal to the data processor 20. The missing measurement indicator 94 can include a single response to find all missing measurements and/or individual responses to find measurements for each missing measurement separately. The confirmation identifier 98 can similarly include a single response to update/store all found measurements and/or selective responses to update/store selected found measurements.

In one instance, the finding can include identifying new and/or additional lesions. For example, measurements are found for a lesion for which no corresponding lesion characteristic information exists in the tracked lesions data store 18. The display can add the lesion with a confirmation to update/store the added lesion and found measurements. In another embodiment, the healthcare practitioner can add a lesion to the tracked lesions 18 via the display and request finding of the missing measurements.

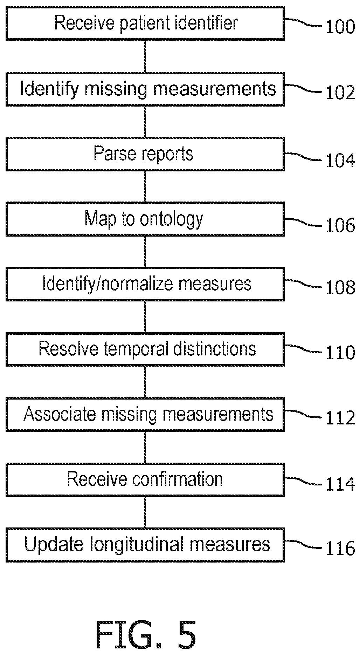

With reference to FIGURE 5, an embodiment of a method of detecting missing measurements with automatic creation of a longitudinal finding view is illustrated. In a step 100, a patient identifier is received. The patient identifier identifies the reports 12 and the tracked lesions 18 corresponding to the patient. The patient identifier can be input by the healthcare practitioner and/or selected from a list of patients.

In a step 102, missing measurements are identified. One or more indicators of the missing measurements are displayed in a constructed display. Temporal identifiers of each report 12 are identified and compared with temporal identifiers of the tracked lesions 18. Missing measurements in the tracked lesions are identified, such as where there exist report temporal identifiers for which measurements according to the corresponding temporal identifier are not present in the tracked lesions. The missing measurements can include multiple temporal identifiers, e.g. measurements missing for more than one temporal identifier of a tracked lesion. The missing measurements can include one or more lesions, e.g. missing for all tracked lesions for a temporal identifier, and/or partial measurements, e.g. missing for some tracked lesions for a temporal identifier. Reports are retrieved from the report data store 12 and tracked lesion data from the tracked lesion data store 18.

In response to receiving a response indicating finding of missing measurements, one or more reports are parsed into sentences in a step 104. Section and paragraph headers are identified. The reports are selected for parsing based on the temporal identifier. In one embodiment, the report with the temporal identifier corresponding to the temporal identifier of each missing measurement is selected. In another embodiment, reports with the temporal identifier corresponding to the temporal identifier of each missing measurement and a subsequent report are selected, e.g. references in subsequent report to confirm measurements and/or confirm identity of the lesion.

In a step 106, phrases of the parsed sentences are mapped to an ontology. For example, headers are mapped and phrases used to determine lesion identities are mapped.

Measurements are identified and normalized in a step 108. The normalized measurements are normalized to the tracked lesion measurements. For example, where tracked lesions measurements are stored in millimeters, measurements in centimeters or inches can be converted to millimeters. Relationships between measurements and the corresponding headers and sentences are preserved, e.g. to which lesion the found measurements correspond.

Temporal distinctions are resolved between measurements in a step 110. Measurements are related to the report temporal identifier or a different report temporal identifier, e.g. cross-report measurement. By analysis of the semantics of the parsed sentences, volumetric information, imaging information, and the like, measurements are identified as corresponding to the report temporal identifier, or corresponding to a prior report temporal identifier. For example, use of words such as "previously" or "previous" can suggest the measurement applies to a different report temporal identifier.

In a step 112, temporally resolved measurements are associated with missing measurements. The association can include displaying the associated or found measurements. For example, the display described in reference to FIGURE 4 is updated with the associated measurements and displayed for healthcare practitioner review. In one embodiment, the display of the associated measurements includes the measurements for one lesion. In one embodiment, the display includes the report fragment, such as the section, paragraph, or sentence with the measurement. The associated measurements can be highlighted, such as high intensity, bold, etc. The display can include the confirmation identifier.

A confirmation is received in a step 114. The confirmation indicates from the healthcare practitioner the tracked lesion data store is to be updated with the associated measurement. The confirmation can include a rejection of the found measurements. In one embodiment, the confirmation includes an option for the healthcare practitioner to enter measurements directly, e.g. report is missing.

In a step 116, the tracked lesion data store 18 is updated with the associated measurements based on the confirmation. The tracked lesion data store 18 can include an identifier of the healthcare practitioner, time stamp, or other data tracking information. The modules can be embodied by or the steps performed by the configured data processor 20.

The above may be implemented by way of computer readable instructions, encoded or embedded on computer readable storage medium, which, when executed by a data processor(s) 20, cause the data processor(s) 20 to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A longitudinal tracking system (10), comprising:
a lesion tracking unit (28), in response to a received patient identifier, being configured to:
construct (102) a display of characteristic information for at least one longitudinally tracked lesion retrieved according to the patient identifier, and an indicator of at least one missing measurement determined by comparing a temporal identifier of retrieved reports with the characteristic information, wherein each report includes a narrative with measurements of at least one reported lesion for the patient identifier; and
a display device (24) configured to display the constructed display of the characteristic information for each longitudinally tracked lesion, and the indicator of the at least one missing measurement.

2. The system (10) according to claim 1, further including:
in response to an indication to find the at least one missing measurement, a document parser engine (30) is configured to parse the narrative of the at least one report and identify section and paragraph headers;
a concept extraction engine (32) configured to map phrases of the parsed sentences to an ontology; and
a measurement engine (34) configured to identify and normalize measurements in the parsed sentences.

3. The system (10) according to claim 2, further including:
a temporal resolution engine (36) configured to identify a temporal identifier for each identified measurement.

4. The system (10) according to claim 3, wherein the identified temporal identifier for each identified measurement includes at least one of the report temporal identifier or a different report temporal identifier.

5. The system according to claim 4, further including:
a control engine (38) configured to associate the identified measurements with the at least one missing measurement based on the identified temporal identifier for each identified measurement and at least one of:
the identified paragraph and section headers;
the mapped phrases;
semantic meaning from the parsed sentences;
measurement comparisons with tracked measurements from a different temporal identifier; or
image references.

6. The system according to any one of claims 1 to 5, wherein the lesion tracking unit (28) is further configured to construct a display of the characteristic information for each longitudinally tracked lesion, and the associated measurements.

7. The system according to any one of claims 1 to 6, wherein the measurement includes a first longest length of a lesion taken from an image slice and a second longest length orthogonal to the first longest length.

8. The system according to either one of claims 6 and 7, wherein the temporal resolution engine (36) is further configured to identify the temporal identifier for one or more referenced images in the narrative based on the narrative.

9. The system according to any one of claims 6 to 8, wherein the lesion tracking unit (28) is further configured to display based on the associated measurements at least one of:
a fragment of the report narrative which includes one of the associated measurements; and
a referenced image corresponding to one of the associated measurements.

10. The system according to any one of claims 6 to 9, wherein the lesion tracking unit (28), in response to an indication confirming update of the at least one longitudinally tracked lesion with the associated measurements, is configured to store the associated measurements and identified temporal identifier in a data store.

11. A method of longitudinal tracking, comprising:
in response to a received patient identifier, displaying (102) on a display device (24) a constructed display of characteristic information for at least one longitudinally tracked lesion retrieved according to the patient identifier, and an indicator of at least one missing measurement determined by comparing a temporal identifier of retrieved reports with the characteristic information, and each report includes a narrative with measurements of at least one reported lesion for the patient identifier.

12. The method according to claim 11, further including:
in response to an indication to find the at least one missing measurement, parsing (104) the narrative of the at least one report into sentences and identifying section and paragraph headers;
mapping (106) phrases of the parsed sentences to an ontology; and
identifying and normalizing (108) measurements in the parsed sentences.

13. The method according to claim 12, further including:
identifying (110) a temporal identifier for each identified measurement.

14. The method according to claim 13, further including:
associating (112) the identified measurements with the at least one missing measurement based on the identified temporal identifier for each identified measurement and at least one of:
the identified paragraph and section headers;
the mapped phrases;
semantic meaning from the parsed sentences;
measurement comparisons with tracked measurements from a different temporal identifier; or
image references.

15. The method according to either one of claims 13 and 14, wherein identifying (112) further includes identifying a temporal identifier for one or more referenced images in the narrative based on the narrative.

## Patentansprüche

1. Längsverfolgungssystem (10), umfassend:
eine Einheit zur Läsionsverfolgung (28), die konfiguriert ist, als Reaktion auf einen empfangenen Patientenbezeichner, zum:
Konstruieren (102) einer Anzeige von charakteristischen Informationen für mindestens eine längs verfolgte Läsion, die gemäß dem Patientenbezeichner abgerufen wurde, und einem Indikator von mindestens einer fehlenden Messung, die bestimmt wurde durch Vergleichen eines temporären Bezeichners von abgerufenen Berichten mit den charakteristischen Informationen, wobei jeder Bericht eine Erzählung mit Messungen von mindestens einer für den Patientenbezeichner berichteten Läsion enthält;
und eine Anzeigevorrichtung (24), die konfiguriert ist zur Anzeige der konstruierten Anzeige der charakteristischen Informationen für jede längs verfolgte Läsion und des Indikators der mindestens einen fehlenden Messung.

2. System (10) nach Anspruch 1, weiter enthaltend:
als Reaktion auf eine Indikation, die mindestens eine fehlende Messung zu finden, eine Dokumentenanalyse-Maschine (30), die konfiguriert ist zum Analysieren der Erzählung des mindestens einen Berichts und Identifizieren von Abschnitts- und Absatzkopfzeilen;
eine Maschine zur Konzeptextraktion (32), die konfiguriert ist zum Zuordnen von analysierten Phrasen zu einer Ontologie; und
eine Messungs-Maschine (34), die konfiguriert ist zum Identifizieren und Normalisieren von Messungen in den analysierten Sätzen.

3. System (10) nach Anspruch 2, weiter enthaltend:
eine Maschine zur temporären Auflösung (36), die konfiguriert ist zum Identifizieren eines temporären Bezeichners für jede identifizierte Messung.

4. System (10) nach Anspruch 3, wobei der identifizierte temporäre Bezeichner für jede identifizierte Messung mindestens eines vom temporären Bezeichner des Berichts oder einem unterschiedlichen temporären Bezeichner des Berichts enthält.

5. System nach Anspruch 4, weiter enthaltend:
eine Steuerungs-Maschine (38), die konfiguriert ist zum Verknüpfen der identifizierten Messungen mit der mindestens einen fehlenden Messung auf der Grundlage des identifizierten temporären Bezeichners für jede identifizierte Messung und mindestens eines von:
den identifizierten Absatz- und Abschnittskopfzeilen;
den zugeordneten Phrasen;
der semantischen Bedeutung aus den analysierten Sätzen;
Messungsvergleichen mit verfolgten Messungen von einem unterschiedlichen temporären Bezeichner; oder
Bildreferenzen.

6. System nach einem der Ansprüche 1 bis 5, wobei die Einheit zur Läsionsverfolgung (28) weiter konfiguriert ist zum Konstruieren einer Anzeige der charakteristischen Informationen für jede längs verfolgte Läsion und der verknüpften Messungen.

7. System nach einem der Ansprüche 1 bis 6, wobei die Messung eine erste längste Länge einer Läsion enthält, die aus einem Bildschnitt entnommen wurde, und eine zweite längste Länge, die zur ersten längsten Länge orthogonal ist.

8. System nach einem der Ansprüche 6 und 7, wobei die Maschine zur temporären Auflösung (36) weiter konfiguriert ist zum Identifizieren des temporären Bezeichners für eines oder mehrere Referenzbilder in der Erzählung auf der Grundlage der Erzählung.

9. System nach einem der Ansprüche 6 bis 8, wobei die Einheit zur Läsionsverfolgung (28) weiter konfiguriert ist zur Anzeige auf der Grundlage der verknüpften Messungen von mindestens einem von:
einem Fragment der Berichterzählung, das eine der verknüpften Messungen enthält; und
einem durch Hinweis gekennzeichneten Bild, das einer der verknüpften Messungen entspricht.

10. System nach einem der Ansprüche 6 bis 9, wobei die Einheit zur Läsionsverfolgung (28) als Reaktion auf eine Indikation, welche die Aktualisierung der mindestens einen längs verfolgten Läsion mit den verknüpften Messungen bestätigt, konfiguriert ist zum Speichern der verknüpften Messungen und des identifizierten temporären Bezeichners in einem Datenspeicher.

11. Verfahren zur Längsverfolgung, umfassend:
als Reaktion auf einen empfangen Patientenbezeichner, Anzeigen (102) auf einer Anzeigevorrichtung (24) einer konstruierten Anzeige von charakteristischen Informationen von mindestens einer längs verfolgten Läsion, die gemäß dem Patientenbezeichner abgerufen wurde, und eines Indikators von mindestens einer fehlenden Messung, die durch Vergleichen eines temporären Bezeichners der abgerufen Berichte mit den charakteristischen Informationen bestimmt wurde, und jeder Bericht eine Erzählung mit Messungen von mindestens einer berichteten Läsion für den Patientenbezeichner enthält.

12. Verfahren nach Anspruch 11, weiter enthaltend:
als Reaktion auf eine Indikation, die mindestens eine fehlende Messung zu finden, Analysieren (104) der Erzählung des mindestens einen Berichts in Sätzen und Identifizieren von Abschnitts- und Absatzkopfzeilen;
Zuordnen (106) von Phrasen der analysierten Sätze zu einer Ontologie; und
Identifizieren und Normalisieren (108) von Messungen in den analysierten Sätzen.

13. Verfahren nach Anspruch 12, weiter enthaltend:
Identifizieren (110) eines temporären Bezeichners für jede identifizierte Messung.

14. Verfahren nach Anspruch 13, weiter enthaltend:
Verknüpfen (112) der identifizierten Messungen mit der mindestens einen fehlenden Messung auf der Grundlage des identifizierten temporären Bezeichners für jede identifizierte Messung und mindestens eines von:
den identifizierten Absatz- und Abschnittskopfzeilen;
den zugeordneten Phrasen;
der semantischen Bedeutung aus den analysierten Sätzen;
Messungsvergleichen mit verfolgten Messungen von einem unterschiedlichen temporären Bezeichner; oder
Bildreferenzen.

15. Verfahren nach einem der Ansprüche 13 und 14, wobei das Identifizieren (112) weiter das Identifizieren eines temporären Bezeichners für eines oder mehrere durch Hinweis gekennzeichnete Bilder in der Erzählung auf der Grundlage der Erzählung enthält.

## Revendications

1. Système de suivi longitudinal (10), comprenant :
une unité de suivi de lésion (28), en réponse à un identifiant de patient reçu, qui est configurée pour :
construire (102) un affichage d'informations caractéristiques pour au moins une lésion longitudinalement suivie récupérée selon l'identifiant du patient et un indicateur d'au moins une mesure manquante déterminée en comparant un identifiant temporel de rapports récupérés aux informations caractéristiques, dans lequel chaque rapport comprend un narratif avec des mesures d'au moins une lésion rapportée pour l'identifiant de patient ; et
un dispositif d'affichage (24) configuré pour afficher l'affichage construit des informations caractéristiques pour chaque lésion longitudinalement suivie et l'indicateur de la au moins une mesure manquante.

2. Système (10) selon la revendication 1, comprenant en outre :
en réponse à une indication permettant de rechercher la au moins une mesure manquante, un moteur d'analyse de document (30) est configuré pour analyser le narratif du au moins un rapport et identifier des en-têtes de sections et de paragraphes ;
un moteur d'extraction de concept (32) configuré pour mapper des expressions des phrases analysées à une ontologie ; et
un moteur de mesure (34) configuré pour identifier et normaliser des mesures dans les phrases analysées.

3. Système (10) selon la revendication 2, comprenant en outre :
un moteur de résolution temporelle (36) configuré pour identifier un identifiant temporel pour chaque mesure identifiée.

4. Système (10) selon la revendication 3, dans lequel l'identifiant temporel identifié pour chaque mesure identifiée comprend au moins l'un de l'identifiant temporel de rapport ou d'un identifiant temporel de rapport différent.

5. Système selon la revendication 4, comprenant en outre :
un moteur de commande (38) configuré pour associer les mesures identifiées à la au moins une mesure montante sur la base de l'identifiant temporel identifié pour chaque mesure identifiée et d'au moins l'un des éléments suivants :
les en-têtes de paragraphes et de sections identifiés ;
les expressions mappées ;
la signification sémantique issue des phrases analysées ;
les comparaisons de mesures à des mesures suivies à partir d'un identifiant temporel différent ; ou
des références d'images.

6. Système selon l'une quelconque des revendications 1 à 5, dans lequel l'unité de suivi de lésion (28) est en outre configurée pour construire un affichage des informations caractéristiques pour chaque lésion longitudinalement suivie et des mesures associées.

7. Système selon l'une quelconque des revendications 1 à 6, dans lequel la mesure comprend une première longueur la plus longue d'une lésion prise dans une tranche d'image et une seconde longueur la plus longueur orthogonale à la première longueur la plus longue.

8. Système selon l'une ou l'autre des revendications 6 et 7, dans lequel le moteur de résolution temporelle (36) est en outre configuré pour identifier l'identifiant temporel pour une ou plusieurs images référencées dans la narratif sur la base du narratif.

9. Système selon l'une quelconque des revendications 6 à 8, dans lequel l'unité de suivi de lésion (28) est en outre configurée pour afficher sur la base des mesures associées au moins l'un des éléments suivants :
un fragment du narratif de rapport qui comprend l'une des mesures associées ; et
une image référencée correspondant à l'une des mesures associées.

10. Système selon l'une quelconque des revendications 6 à 9, dans lequel l'unité de suivi de lésion (28), en réponse à une indication confirmant la mise à jour de la au moins une lésion longitudinalement suivie avec les mesures associées, est configurée pour stocker les mesures associées et l'identifiant temporel identifié dans une mémoire de données.

11. Mesure de suivi longitudinal comprenant :
en réponse à un identifiant de patient reçu, l'affichage (102) sur un appareil d'affichage (24) d'un affichage construit d'informations caractéristiques pour au moins une lésion longitudinalement suivie récupérée selon l'identifiant du patient et un indicateur d'au moins une mesure manquante déterminée en comparant un identifiant temporel de rapports récupérés aux informations caractéristiques et chaque rapport comprend un narratif avec des mesures d'au moins une lésion rapportée pour l'identifiant de patient.

12. Procédé selon la revendication 11, comprenant en outre :
en réponse à une indication pour trouver la au moins une mesure manquante, l'analyse (104) du narratif du au moins un rapport en phrases et l'identification d'en-têtes de sections et de paragraphes ;
le mappage (106) d'expressions de phrases analysées à une ontologie ; et
l'identification et la normalisation (108) de mesures dans les phrases analysées.

13. Procédé selon la revendication 12, comprenant en outre :
l'identification (110) d'un identifiant temporel pour chaque mesure identifiée.

14. Procédé selon la revendication 13, comprenant en outre :
l'association (112) des mesures identifiées à la au moins une mesure manquante sur la base de l'identifiant temporel identifié pour chaque mesure identifiée et d'au moins l'un des éléments suivants :
les en-têtes de paragraphes et de sections identifiés ;
les expressions mappées ;
la signification sémantique issue des phrases analysées ;
les comparaisons de mesures avec des mesures suivies issues d'un identifiant temporel différent ; ou
des références d'images.

15. Procédé selon l'une ou l'autre des revendications 13 ou 14, dans lequel l'identification (112) comprend en outre l'identification d'un identifiant temporel pour une ou plusieurs images référencées dans le narratif sur la base du narratif.
